# EUROPEAN PATENT APPLICATION

(11) **EP 4 759 310 A2**
(43) Date of publication of application: **17.06.2026**
(21) Application number: 25224664.0
(22) Date of filing: 17.12.2025
(51) Int. Cl.: A61K 31/198, A61K 9/00, A61K 31/405, A61K 31/4402, A61K 31/455, A61K 31/47, A61P 31/10

(54) **TRYPTOPHAN OR ITS METABOLITE FROM THE KYNURENINE PATHWAY FOR USE IN THE PREVENTION OR TREATMENT OF DERMATOPHYTOSIS**

(30) Priority: 16.12.2024 PL 45060624
(71) Applicant: Uniwersytet Lodzki, 90-136 Lodz (PL); Katolicki Uniwersytet Lubelski Jana Pawla II, 20-059 Lublin (PL); Uniwersytet Medyczny w Lublinie, 20-059 Lublin (PL)
(72) Inventor: Ciesielska, Anita, 95-030 Starowa Gora (PL); Dzitko, Katarzyna, 95-010 Dobieszkow (PL); Straczek, Pawel, 95-010 Swedow (PL); Turski, Waldemar, 20-868 Lublin (PL); Turska-Kozlowska, Monika, 20-882 Lublin (PL); Sobczynski, Jan, 20-802 Lublin (PL)
(74) Representative: Kicinska-Fujawa, Alicja

(57) **Abstract**

The object of the invention is tryptophan or metabolite thereof from the kynurenine pathway selected from a group comprising 3-hydroxykynurenin, quinolinic acid, kynurenic acid, quinaldic acid and picolinic acid for use in the prevention or treatment of dermatophytoses, especially caused by *Trichophyton rubrum* and composition, preferably in the form of nail polish.

## Description

The subject of the invention is tryptophan or metabolite thereof from the kynurenine pathway, for use in the prevention or treatment of *dermatophytoses,* caused specifically by *Trichophyton rubrum.*

*Trichophyton rubrum (T. rubrum)* is the most common anthropophilic dermatophyte found on human skin, responsible for more than 60% of all superficial mycoses in humans. It causes fungal infections of the nails and feet, as well as the smooth skin and groin. Unlike other fungi, dermatophytes can attack healthy people with normally functioning immune systems. The fungus is transmitted through human-to-human contact with a carrying person, and infection with this dermatophyte is a worldwide problem.

Currently, topical or systemic medications are used in dermatophytoses. Topical medications recommended and most commonly used for onychomycosis (nail infections) include ciclopirox and amorolfine in nail polish form. In mycoses of feet, groin and smooth skin, topical use involves imidazole derivatives in the form of creams, solutions and gels (e.g. clotrimazole, ketoconazole), allylamines in the form of creams (e.g. terbinafine), amorolfine in cream and ciclopirox in the form of cream, gel or suspension.

Oral itraconazole, fluconazole and terbinafine are used for systemic treatment in all of the above-mentioned dermatophytoses. Pulsed (cyclic) method for using the medications is recommended [1].

Despite the wide availability of antifungal medications, the treatment of superficial mycoses remains a challenge due to the need for systematic, long-term therapy, the location of lesions in poorly vascularised areas, which significantly hinders drug penetration and its efficacy, as well as the development of dermatophyte tolerance and resistance to available medications [2].

Combined treatment is recommended for patients with extensive mycoses, the elderly, and those with previous treatment being unsuccessful. Combination treatment may involve use of two different systemic medications, in separate pulses following sequentially, or using systemically administered medications with the concomitant use of topical medications [3].

Tryptophan is a building-block amino acid for living organisms. In case of humans, tryptophan is an essential amino acid that is supplied to the body with food. In contrast, fungi, including *T. rubrum,* produce tryptophan exogenously [4]. In addition to its building function in protein synthesis, tryptophan undergoes numerous metabolic transformations in the indole, serotonin, and kynurenine pathways [5]. Tryptophan metabolism in the dermatophyte *T. rubrum* is unknown.

The term "kynurenine pathway" is used to describe the tryptophan's metabolic cycle. The pathway start with tryptophan conversion to kynurenine. The main pathway leads, through subsequent enzymatic transformations to quinolinic acid formation, which is the precursor of Nicotinamide Adenine Dinucleotide (NAD). Tryptophan is converted to kynurenine under the influence of the enzymes: tryptophan 2,3-dioxygenase (TDO) (EC 1.13.11.11) and indoleamine 2,3-dioxygenase (IDO) (EC 1.13.11.52). Kynurenine is converted by kynurenine 3-monooxygenase (KMO) (EC 1.14.13.9) to 3-hydroxykynurenine. 3-Hydroxykynurenine is converted to 3-hydroxyanthranilic acid under the influence of kynureninase (EC 3.7.1.3). From it, under the influence of 3-hydroxyanthranilate 3,4-dioxygenase (EC 1.13.11.6), an intermediate compound called 2-amino-3-carboxymuconic semialdehyde is formed, which spontaneously converts to quinolinic acid. The side paths of the kynurenine pathway also produce anthranilic acid under the influence of kynureninase, and kynurenic acid, as well as xanthurenic acid under the influence of kynurenine aminotransferase, which occurs in 4 isoforms. In addition, cinnabaric acid and spontaneously picolinic acid are formed as a result of autooxidation. The processes described have been defined in animals [6].

As used in this description, the terms "treat" or "treatment" refer to means of relieving or removing a disease, or its accompanying symptoms. The term "prevention" refers to a method of reducing the likelihood, or eliminating the possibility of developing a disorder.

As used herein, the expression "dermatophytosis", also known as ringworm, refers to a skin infection caused by fungi of the dermatophyte group.

To date, the state of the art has known uses for tryptophan or its metabolites from the kynurenine pathway.

Tryptophan has the molecular formula C₁₁H₁₂N₂O₂; PubChem CID 6305. L-tryptophan enantiomer is used as a supplement taken orally. It is intended to facilitate falling asleep and improve mood, probably through its metabolites: serotonin and melatonin. Bioactive cosmetic compositions containing tryptophan are disclosed in the patent PL227196. On the other hand, surfactant compounds as cationic salts of tryptophan and the method of producing them are described in the patent PL230166. Based on an *in vitro* study conducted on keratinocytes, it has been demonstrated that tryptophan can accelerate skin wound healing. In a clinical trial, 1% tryptophan gel was applied externally to skin wounds in humans and was found to accelerate wound healing and reduce pain sensation in patients [7]. Tryptophan stimulated *in vitro* proliferation of the ocular corneal epithelium, and showed no toxic effects when administered to rabbit eye surface [8].

Quinolinic acid (pyridine-2,3-dicarboxylic acid) has a total molecular formula: C₇H₅NO₄, PubChem CID 1066. Quinolinic acid is a neurotoxin [9]. In contrast, little is known about its role outside the central nervous system. Its urinary levels have been found to increase in numerous health disorders [10]. A method for producing quinolinic acid using a recombinant microorganism is disclosed in the European patent EP3168293.

3-Hydroxykynurenine has a total molecular formula: C₁₀H₁₂N₂O₄, PubChem CID 89. 3-Hydroxykynurenine increases free radical release, which is responsible for its adverse effects in the body. It is considered a neurotoxin. It acts as a UV filter in the in eye [11].

Kynurenic acid has a molecular formula: C₁₀H₇NO₃, PubChem CID 3845. Kynurenic acid demonstrates neuroprotective and anticonvulsant effects in the brain, and also has antiinflammatory and antioxidant effects. It has antiatherosclerotic, analgesic, anti-ulcer, hepatic and pancreatic protective effects (see patent PL228037), reduces body mass gain (see patent PL239745), regulates lipid profile, increases glucose tolerance. It demonstrates antibacterial, antiviral and antineoplastic activity. When applied topically, it accelerates corneal defect healing and influences skin healing by reducing scarring [12] (see patent PL237591). The patent PL237892 discloses a pharmaceutical composition based on kynurenic acid and ethanol, for internal use in droplet form, intended for prevention or reduction of overweight, or obesity. Patent PL234304, on the other hand, discloses the medical use of kynurenic acid, which causes the heart rate decrease, for producing a medication to treat and prevent disorders characterised by heart rate increase. While, the patent PL233551 discloses kynurenic acid use to treat and prevent the formation and dissolution of deposits and stones, e.g. in biliary tract.

The anti-fibrotic effects of kynurenine and its metabolites, including kynurenic acid, were disclosed in the international application WO 2014/194407.

Xanthurenic acid (4,8-dihydroxyquinoline-2-carboxylic acid) has a molecular formula: C₁₀H₇NO₄, PubChem CID 5699. Xanthurenic acid is considered to be a type II metabotropic glutamate receptor agonist. It affects glutamate transport mechanisms across cell membranes. There are suggestions of its involvement in the pathomechanism of schizophrenia. Xanthurenic acid is a sepiapterin reductase inhibitor [13].

Quinaldic acid (known as quinoline-2-carboxylic acid) has a molecular formula: C₁₀H₇NO₂, PubChem CID 7124. Knowledge of quinaldic acid's biological significance is scarce. Its effects on liver enzyme activity, insulin synthesis and gluconeogenesis processes, as well as its antiproliferative effects, have been described [14].

Picolinic acid (pyridine-2-carboxylic acid) has a molecular formula: C₆H₅NO₂, PubChem CID 1018. Picolinic acid has chelating properties, and is sometimes used in combination with chromium and iron to treat diabetes and anaemia. It has inconclusive effects on nerve cell excitability; it shows a proconvulsant effect, yet attenuates the neurotoxic effects of quinolinic acid [15]. The patent PL203187 discloses an oral composition containing picolinic acid and peroxide particles for use in the treatment of a condition selected from the group comprising teeth whitening, actions against plaque, oral odour, caries and calculus.

Despite the numerous applications of tryptophan or its metabolites from the kynurenine pathway disclosed in the state of the art, there is no information in the available literature regarding the use of tryptophan, quinolinic acid, 3-hydroxykynurenine, kynurenic acid, xanthurenic acid, quinaldic acid or picolinic acid in the treatment of dermatophytoses in humans and animals.

The technical problem challenged by the present invention is to provide prevention or treatment of mycoses caused by *Trichophyton rubrum.*

Unexpectedly, during the inventors' screening directed at finding antifungal substances, tryptophan or its metabolite from the kynurenine pathway, including quinolinic acid, 3-hydroxykynurenine, kynurenic acid, xanthurenic acid, quinaldic acid, picolinic acid, were found to inhibit *T. rubrum* growth.

The subject of the invention is tryptophan or a metabolite thereof from the kynurenine pathway, selected from the group comprising 3-hydroxykynurenine, quinolinic acid, kynurenic acid, xanthurenic acid, quinaldic acid and picolinic acid for use in the prevention or treatment of dermatophytoses, particularly those caused by *Trichophyton rubrum.*

As shown in the embodiments, depending on the substance and its concentration, it has a growth-reducing or growth-inhibiting effect on *Trichophyton rubrum.* Among the substances tested, the highest antifungal activity was demonstrated by quinolinic acid, which inhibited *Trichophyton rubrum* growth in the concentration range from 0.625 mM. On the other hand, quinaldinic acid and picolinic acid showed a growth-limiting effect on *T. rubrum* at a concentration of 50 mM. Thus, according to the invention, for the purposes of achieving the desired fungistatic or fungicidal effect, tryptophan or its metabolite from the kynurenine pathway, or a combination thereof, may be used at the minimum concentration considered effective, and with pharmaceutically acceptable excipients.

Since *Trichophyton rubrum* causes onychomycosis of fingernails and toenails, *tryptophan* or a metabolite thereof from the kynurenine pathway, selected from the group comprising 3-hydroxykynurenine, quinolinic acid, kynurenic acid, xanthurenic acid, quinaldic acid and picolinic acid for use according to the invention, is administered topically, preferably in the form of a nail polish that can be applied to the affected nails.

The composition of the medicinal nail polish consists of a base in which an active substance with antifungal activity is dissolved or suspended. The penetrability of an active substance is inversely proportional to its molecular weight, and also depends on active substance ionisation, the wetting angle of the preparation, the liquid's adhesion on the nail plate surface, and the fineness level of the active substance that is suspended in the base. Therefore, the nail polish should exhibit adhesion to allow prolonged activity on the nail surface, and easy wettability resulting in good plate coverage. In addition, the nail polish composition should have a low viscosity, low surface tension.

In the field of therapeutic nail formulations, the prior art describes anhydrous, hydrophilic and oil based nail polish compositions.

The anhydrous nail polishes preferably contain: organic solvents such as ethyl acetate, butyl acetate, anhydrous ethyl alcohol, isopropyl alcohol, triacetin, thickening agents, e.g. cetostearyl alcohol, and components forming a film on the nail surface, such as methylvinyl ether copolymer with monobutyl maleate (1:1), ammonium methacrylate copolymer (type A), methoxyethene polymer with 2-butenedioic acid monobutyl ester, etc.

Hydrophilic nail polishes, on the other hand, preferably contain: polar solvents such as water, propylene glycol, ethyl alcohol, viscosity enhancers: hydroxypropyl cellulose, hydroxypropyl methylcellulose, nail plate penetration enhancers: carbocysteine, N-acetylcysteine, lactic acid, polyoxoethylene glycol 300 or 400, sodium dodecyl sulphate, polyethyleneglycol 16 oleyl or linoleyl ether, polyethyleneglycol 17 glyceride of caprylic and caproic acids, wetting agents: cyclomethicone, dimethicone, surface tension reducing agents, such as myristyl lactate, diisopropyl adipate, solubilisers such as Polyoxyl 40 hydrogenated castor oil, beta-cyclodextrin, hydroxypropyl beta-cyclodextrin, preservatives, pH modifiers such as sodium hydroxide, hydrochloric acid).
- The formulation of an anhydrous nail polish for use according to the invention may be either an anhydrous nail polish, with the composition shown in Table 1, or a hydrophilic nail polish, with the composition shown in Table 2, the active substance in each composition variant being any of the following substances comprising tryptophan or a metabolite thereof from the kynurenine pathway, meaning:
- tryptophan, molecular formula C₁₁H₁₂N₂O₂, CAS number 73-22-3;
- quinolinic acid [pyridine-2,3-dicarboxylic acid], molecular formula C₇H₅NO₄, CAS number 89-00-9;
- 3-hydroxykynurenine [3-hydroxykynurenine], molecular formula C₁₀H₁₂N₂O₄, CAS number 2147-61-7;
- kynurenic acid, molecular formula C₁₀H₇NO₃, CAS number 492-27-3.
- quinaldic acid [quinoline-2-carboxylic acid], molecular formula C₁₀H₇NO₂, CAS 1580002-30-7;
- picolinic acid [pyridine-2-carboxylic acid], molecular formula C₆H₅NO₂, CAS 98-98-6.

**Table 1 Waterless nail polish composition**

| **Substance** | **CAS No** | **Concentration [wt%]** |
|---|---|---|
| Active substance | | 1-5 |
| Ethyl alcohol | 64-17-5 | 45-50 |
| Butyl acetate | 123-86-4 | 10 |
| Ethyl acetate | 141-78-6 | 35 |
| Triacetin | 102-76-1 | 2 |
| Ammonium methacrylate copolymer (Type A) | 33434-24-1 | 2 |

**Table 2 Hydrophilic nail polish composition**

| **Substance** | **CAS No** | **Concentration [wt%]** |
|---|---|---|
| Active substance | | 1-5 |
| Polyoxyl 40 hydrogenated castor oil | 61788-85-0 | 5-9 |
| Ethyl alcohol | 64-17-5 | 20 |
| Propylene glycol | 57-55-6 | 2 |
| Low viscosity Hydroxypropyl methylcellulose | 9004-65-3 | 1 |
| Dimethicone | 9006-65-9 | 0.5 |
| Hydrochloric acid | 7647-01-0 | 0.05 |
| Purified water | 7732-18-5 | 66.45 |

As it is the practice in the topical treatment of mycoses, especially refractory mycoses, to use compositions containing combinations of different antifungal agents in order to increase their efficacy, tryptophan or its metabolite from the kynurenine pathway selected from the group comprising 3-hydroxykynurenine, quinolinic acid, kynurenic acid, quinaldic acid and picolinic acid can also be used in compound compositions for topical application in combination with other antifungal agents.

For particularly resistant mycoses, it is medical practice to use topically acting medications and systemically acting medications, therefore tryptophan or a metabolite thereof from the kynurenine pathway selected from a group comprising 3-hydroxykynurenine, quinolinic acid, kynurenic acid, quinaldic acid and picolinic acid can be used in combination treatment, involving using a combination of topically acting medications, such as a nail polish, and systemically acting medications, such as a tablet.

As antifungal agents acting against *Trichophyton rubrum* are also effective against dermatoses caused by other fungi, tryptophan or a metabolite thereof from the kynurenine pathway can be used to prevent infection and treat diseases caused by other fungi.

In the detailed example of the invention presented below, a description of the experimental work carried out by the inventors is provided, whereby the results presented do not limit the invention.

### EXAMPLE 1

### Evaluation of antifungal activity

Antifungal activity test of tryptophan and kynurenine metabolites was carried out on a reference strain of *Trichophyton rubrum,* from the Dutch collection of the Westerdijk Fungal Biodiversity Institute, deposited under No. CBS 120358.

The antimicrobial activity of tryptophan and kynurenine metabolites was examined by determining the minimum inhibitory concentration, MIC (Minimal Inhibitory Concentration) by serial dilutions in a 96-well plate, based on the EUCAST standard [16].

*Trichophyton rubrum* CBS 120358 cultures (14 - 21 days old) on slants with solid Sabouraud medium were washed with 5 mL of a 0.1% sterile distilled water solution with Tween 20. The mycelium was then harvested with a sterile inoculation loop and aspirated through a filter (Corning Sterile Cell Strainer, 40 µm) into a 50 mL Falcon tube and centrifuged (5 minutes, 4°C, 5000 rpm). The supernatant was removed and the fungal spores were inoculated with YG (Yeast Extract Glucose) medium in a volume of 20 mL. After two hours of incubation with shaking (37°C, 140 rpm), the germinated spores together with the medium were transferred to a new Falcon tube (V = 50 mL) and centrifuged (5 minutes, 4°C, 5000 rpm). After supernatant removal, the precipitate was suspended in 2 mL of sterile distilled water and transferred to a glass test tube with a stopper. According to the EUCAST standard, an alternative method was used to establish a cell density of 2 - 5 × 10⁶ CFU/mL by densitometric determination of a cell suspension density of 0.5 on the McFarland scale. The suspension thus prepared was again diluted 1:10 to obtain inoculating material of 2 - 5 ×¹⁰⁵ CFU/ml and added to the wells of the 96-well plate within 30 minutes.

The tested substances tryptophan (L-tryptophan), 3-hydroxykynurenine (3-hydroxy-DL-kynurenine), quinolinic acid (2,3-pyridinedicarboxylic acid), kynurenic acid (kynurenic acid), xanthurenic acid, quinaldic acid, picolinic acid (2-picolinic acid) were sourced from certified high-purity chemical reagent supplier Sigma-Aldrich.

Test substance solutions were prepared in RPMI 1640 liquid medium (Gibco), containing 2% w/v glucose (Sigma-Aldrich), obtaining the following concentrations: 0.625; 1.25; 2.5; 5.0; 10.0; 12.5; 25.0; 50.0 mM. The positive control for the test (fungal growth) was the test medium (RPMI 1640 liquid medium, containing 2% w/v glucose) and the negative control for the test (no fungal growth) was the antibiotic amphotericin B (Sigma-Aldrich), at 4 µg/mL concentration (4.33 mM). The experiment was conducted in 3 series of 3 repetitions. The results obtained for all three repetitions were the same. MIC values were determined based on microscopic observations of morphological changes in *Trichophyton rubrum* induced by the metabolites. The following were assessed for the cultures: the presence of hyphae and spores of the fungus. The presence of fungal hyphae in the field of view was indicative of *Trichophyton rubrum* growth and indicated absence of antifungal activity of the tested compound, whereas the presence of *Trichophyton* rubrum spores in the field of view indicated absence of *Trichophyton rubrum* growth and indicated an antifungal effect of the test compound.

The results of the antifungal activity for the tested substances are shown in Table 1.

**Table 1.**

| Substance | **Substance Concentration [mM]** | | | | | | | | |
|---|---|---|---|---|---|---|---|---|---|
| | 0 | 0.625 | 1.25 | 2.5 | 5 | 10 | 12.5 | 25 | 50 |
| Tryptophan | + | + | + | + | + | + | + | + | - |
| Quinolinic Acid | + | - | - | - | - | - | - | - | - |
| 3-Hydroxykynurenine | + | + | + | + | - | - | nt | nt | nt |
| Kynurenic acid | + | + | + | + | + | + | + | + | - |
| Xanthurenic Acid | + | + | + | + | +/- | +/- | - | - | - |
| Quinaldic acid | + | + | + | + | + | + | + | + | +/- |
| Picolinic acid | + | + | + | + | + | + | + | + | +/- |
| Amphotericin B | - | | | | | | | | |

| | | | | | | | | | |
|---|---|---|---|---|---|---|---|---|---|
| Legend: the "+" symbol indicates the presence of *Trichophyton rubrum* hyphae in the field of view, and indicates fungal growth; the "+/-" symbol indicates the presence of *Trichophyton rubrum* hyphae and spores in the field of view, and indicates limited growth of the fungus. the "-" symbol indicates the presence of spores and the absence of *Trichophyton rubrum* hyphae in the field of view, and indicates the absence of fungal growth; "nt" means that no test was carried out at this concentration. | | | | | | | | | |

### RESULTS

Among the substances tested, the highest antifungal activity was demonstrated by quinolinic acid, which inhibited *Trichophyton rubrum* growth in the concentration range from 0.625 mM. In contrast, at a concentration of 5 mM, 3-hydroxykynurenine inhibits fungal growth, and xanthurenic acid reduces its growth, while at concentrations of 12.5 mM and higher, xanthurenic acid also inhibited the *Trichophyton rubrum* growth. On the other hand, at a concentration of 50 mM, tryptophan and kynurenic acid inhibited fungal growth, while the growth-limiting effect on *T. rubrum* was shown by quinaldinic acid and picolinic acid.

### EXAMPLE 2

### Compositions for topical use

In order to obtain the foregoing compositions in a rotary mixer adapted for use with flammable and explosive solvents, a mixing process typical of obtaining therapeutic anhydrous nail polishes was carried out. First, a mixture of solvents (ethyl alcohol, butyl acetate, ethyl acetate, triacetin) was prepared by stirring at 300 rpm over 10 minutes, followed by the dispersion of ammonium methacrylate copolymer (type A) and the blend was thoroughly mixed for 30 minutes at 500 rpm. Finally, the active substance was added and stirred for 10 minutes at 750 rpm.

Hydrophilic nail polish compositions with the following ingredients were prepared.

| **Substance** | **CAS No** | **Concentration [wt%]** | **Concentration [wt%]** |
|---|---|---|---|
| | | Composition 1 | Composition 2 |
| Active substance | | 1 | 5 |
| Polyoxyl 40 hydrogenated castor oil | 61788-85-0 | 5 | 9 |
| Ethyl alcohol | 64-17-5 | 20 | 20 |
| Propylene glycol | 57-55-6 | 2 | 2 |
| Low viscosity Hydroxypropyl methylcellulose | 9004-65-3 | 1 | 1 |
| Dimethicone | 9006-65-9 | 0.5 | 0.5 |
| Hydrochloric acid | 7647-01-0 | 0.05 | 0.05 |
| Purified water | 7732-18-5 | 70.45 | 62.45 |
| Composition pH | | 5.5 | 3.5 |

The active substance was a substance selected from a group comprising: tryptophan, 3-hydroxykynurenine, quinolinic acid, kynurenic acid, quinaldic acid and picolinic acid.

To obtain the above-mentioned composition, a mixture of solvents (water, glycol and ethanol) was prepared in a rotary mixer equipped with an overhead anchor stirrer, then the active substance was added and the entirety was mixed. In the next step, Polyoxyl 40 hydrogenated castor oil, low-viscosity hydroxypropyl methylcellulose, and dimethicone were added successively, and the blend was thoroughly mixed. Finally, hydrochloric acid (as a 28-36% w/w solution) was added until a pH between 3.5 and 5.5 was reached.

In conclusion, the results obtained indicate that tryptophan or the tested kynurenine metabolites reduce or inhibit the growth of the fungus *Trichophyton rubrum,* and can therefore be used in the prevention or treatment of an infection caused by this fungus. As it is the practice in the topical treatment of refractory mycoses to use products containing antifungal agents in combination, in order to increase the efficacy, tryptophan or kynurenine metabolites can be used together with other antifungal agents, in a compound composition for external use. In case of particularly resistant mycoses, the practice is to use topical and systemic medications, therefore tryptophan or kynurenine metabolites can be used in combination treatment involving combined use of topical and systemic medications.

### BIBLIOGRAPHY

**[1]** Maleszka R, Adamski Z, Szepietowski J, Baran E. Treatment of superficial fungal infections-recommendations of experts of Mycological Section of Polish Dermatological Society. Dermatology Review/Przeglad Dermatologiczny. 2015;102(4):305-15 doi: 10.5114/dr.2015.53418
**[2]** Martinez-Rossi NM, Bitencourt TA, Peres NTA, Lang EAS, Gomes EV, Quaresemin NR, Martins MP, Lopes L, Rossi A. Dermatophyte Resistance to Antifungal Drugs: Mechanisms and Prospectus. Front Microbiol. 2018;9:1108. doi: 10.3389/fmicb.2018.01108;
**[3]** See [1];
**[4]** Wang et al., Analysis of the dermatophyte Trichophyton rubrum expressed sequence tags. BMC Genomics 2006, 7:255 doi:10.1186/1471-2164-7-255;
[5] https://encyclopedia.pub/entry/27119;
**[6]** Mithaiwala MN, Santana-Coelho D, Porter GA, O'Connor JC. Neuroinflammation and the Kynurenine Pathway in CNS Disease: Molecular Mechanisms and Therapeutic Implications. Cells. 2021;10(6):1548. doi: 10.3390/cells10061548;
**[7]** Barouti N, Mainetti C, Fontao L, Sorg O. L-Tryptophan as a Novel Potential Pharmacological Treatment for Wound Healing via Aryl Hydrocarbon Receptor Activation. Dermatology. 2015;230(4):332-9. doi: 10.1159/000371876
**[8]** Matysik-Woźniak A, Turski WA, Turska M, Paduch R, ańcut M, Piwowarczyk P, Czuczwar M, Rejdak R. Tryptophan as a Safe Compound in Topical Ophthalmic Medications: In Vitro and In Vivo Studies. Ocul Immunol Inflamm. 2022;30(4):940-950. doi: 10.1080/09273948.2020.1856883
**[9]** Lugo-Huitrón R, Ugalde Muñiz P, Pineda B, Pedraza-Chaverrí J, Ríos C, Pérez-de la Cruz V. Quinolinic acid: an endogenous neurotoxin with multiple targets. Oxid Med Cell Longev. 2013;2013:104024. doi: 10.1155/2013/104024
**[10]** Saade MC, Clark AJ, Parikh SM. States of quinolinic acid excess in urine: A systematic review of human studies. Front Nutr. 2022 Dec 16;9:1070435. doi:10.3389/fnut.2022.1070435. PMID: 36590198
**[11]** Colín-González AL, Maldonado PD, Santamaría A. 3-Hydroxykynurenine: an intriguing molecule exerting dual actions in the central nervous system. Neurotoxicology. 2013 Jan;34:189-204. doi: 10.1016/j.neuro.2012.11.007
**[12]** Turska M, Paluszkiewicz P, Turski WA, Parada-Turska J. A Review of the Health Benefits of Food Enriched with Kynurenic Acid. Nutrients. 2022;14(19):4182. doi: 10.3390/nu14194182
**[13]** Fazio F, Lionetto L, Curto M, lacovelli L, Copeland CS, Neale SA, Bruno V, Battaglia G, Salt TE, Nicoletti F. Cinnabarinic acid and xanthurenic acid: Two kynurenine metabolites that interact with metabotropic glutamate receptors. Neuropharmacology. 2017;112(Pt B):365-372. doi: 10.1016/j.neuropharm. 2016.06.020
**[14]** Nowicka-St 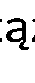 żka P, Langner E, Turski W, Rzeski W, Parada-Turska J. Quinaldic acid in synovial fluid of patients with rheumatoid arthritis and osteoarthritis and its effect on synoviocytes in vitro. Pharmacol Rep. 2018;70(2):277-283. doi: 10.1016/j.pharep.2017.09.010
**[15]** Cioczek-Czuczwar A, Czuczwar P, Turski WA, Parada-Turska J. Influence of picolinic acid on seizure susceptibility in mice. Pharmacol Rep. 2017;69(1):77-80. doi: 10.1016/j.pharep.2016.10.009
**[16]** EUCAST E.DEF 9.4 March 2022, Method for the determination of broth dilution minimum inhibitory concentrations of antifungal agents for conidia forming moulds.

## Claims

1. Tryptophan or a metabolite thereof from the kynurenine pathway, selected from the group comprising 3-hydroxykynurenine, quinolinic acid, xanthurenic acid, kynurenic acid, quinaldic acid and picolinic acid, for use in the prevention or treatment of dermatophytoses, specifically those caused by *Trichophyton rubrum.*

2. Tryptophan or a metabolite thereof from the kynurenine pathway, for use according to claim 1, at a minimum recognised effective concentration and with pharmaceutically acceptable excipients, wherein the minimum recognised effective concentration is:
a. 0.625 mM for quinolinic acid;
b. 5 mM for 3-hydroxykynurenine and xanthurenic acid;
c. 50 mM for tryptophan, kynurenic acid, quinaldic acid and picolinic acid.

3. Tryptophan or a metabolite thereof from the kynurenine pathway, for use according to claim 1, wherein it is administered topically, preferably in the form of a nail polish.
